# NEUE EUROPÄISCHE PATENTSCHRIFT

(11) **EP 0 181 455 B2**
(45) Veröffentlichungstag und Bekanntmachung des Hinweises auf die Entscheidung über den Einspruch: **31.05.2000**
(45) Hinweis auf die Patenterteilung: 21.08.1991
(21) Anmeldenummer: 85111184.9
(22) Anmeldetag: 04.09.1985
(51) Int. Cl.: A61K 38/21, A61K 45/06

(54) **Verwendung von Interferon-gamma (IFN-gamma) enthaltenden Präparationen zur systemischen Behandlung von verschiedenen Erkrankungen des Menschen in niedriger Dosierung**
Use of preparations containing interferon-gamma (IFN-gamma) for systematically treating various human diseases at a low dosage
Application de préparations contenant de l'interféron-gamma (IFN-gamma) pour le traitement systématique de différentes maladies humaines à dose réduite

(30) Priorität: 05.10.1984 DE 3436637; 05.10.1984 DE 3436638; 18.06.1985 DE 3521733
(43) Veröffentlichungstag der Anmeldung: 21.05.1986
(73) Patentinhaber: Boehringer Ingelheim International GmbH, 55216 Ingelheim am Rhein (DE)
(72) Erfinder: Brzoska, Josef, Dr., D-7958 Laupheim (DE); v. Eichborn, Johann-Friedrich, Dr., D-7901 Hüttisheim (DE); Obert, Hans-Joachim, Dr., D-7958 Laupheim (DE)

(56) Entgegenhaltungen:
- EP-A- 0 077 063
- EP-A- 0 080 032
- EP-A- 0 087 686
- EP-A- 0 088 540
- EP-A- 0 107 498
- EP-A- 0 109 234
- EP-A- 0 117 470
- EP-A- 0 119 757
- EP-A- 0 128 009
- EP-A- 0 131 789
- EP-A- 0 149 551
- EP-A- 0 168 214
- EP-A- 0 170 843
- EP-B- 0 077 670
- WO-A-83/01198
- DE-A- 3 001 585
- GB-A- 2 040 292
- US-A- 4 507 281
- "Interferon, "In vivo and clinical studies", Band 4, 1985, Kapitel 20, Herausgeber: N.B. FINTER et al., Elsevier Science Publishers B.V., Amsterdam, NL; S.A. SHERWIN: "The potential clinical applications of gamma-interferon"
- BIOLOGICAL ABSTRACTS, Band 79, Nr. 2, 1985, Seite AB-651, Zusammenfassung Nr. 15031, Philadelphia, PA, US; J.U. GUTTERMAN et al.: "Pharmacokinetic study of partially pure gamma-interferon in cancer patients", & CANCER RES. 44(9): 4164-4171, 1984
- BIOLOGICAL ABSTRACTS, Band 80, Nr. 5, 1985, Seite AB-620, Zusammenfassung Nr. 42174, Philadelphia, PA, US; R. KURZROCK et al.: "Pharmacokinetics, single-dose tolerance and biological activity of recombinant gamma-interferon in cancer patients", & CANCER RES. 45(6): 2866-2872, 1985
- GUTTERMANN et al.: "Pharmacokinetic Study of Partially Pure Gamma-Interferon in Cancer Patients", Cancer Res. 44, p. 4164-4171 (1984)
- SHERWIN, S.A., et al.: "A preliminary phase I trial of partially purified Interferon-gamma in patients with cancer", J. Biol.Resp.Mod., 1984, p. 599-607
- AULITZKY, W. et al.: "Successful treatment of metastatic renal cell carcinoma with a biologically active dose of recombinant interferon-gamma", Journal of Clinical Oncology, vol. 7, no. 12, 1989, pp. 1875-1884
- The Biology of the Interferon System, 1983; K. OSTHER et al., S. 527-533
- Proc.Am.Soc.Clin.Oncol.2, S.46; H.A. HARVEY et al., ASCO ABSTRACTS, 1983, C-177
- V. CARRERO et al.: "A Pilot Study of Reconbinant Gamma-Interferon Treatment of Chronic Hepatitis B", Abstract II-81 eines Vortrags
- J.A. GEORGIADES et al., 1984, "Interferons and their applications", Kap. 15, S. 305-337
- Donna S. STONE-WOLFF et al., 1984; J.Exp.Med. 159, S. 828-843
- Ogawa et al., (March 1985), Proc. Am. Soc. Clin. Oncol. 4, S. 219

## Beschreibung

Alle bekannten Interferone werden auf Grund von Homologien sowohl zwischen den Nukleotidsequenzen ihrer Strukturgene als auch zwischen ihren Aminosäuresequenzen eindeutig in drei Gruppen eingeteilt, die als IFN-alpha, IFN-beta und IFN-gamma bezeichnet werden.

Die verwendeten Bezeichnungen folgen der neuesten Empfehlung des "Interferon Nomenclature Commitee", die von J. Vilcek 1983 in Archives of Virology Vol. 77, S. 283-285, veröffentlicht wurden. Zusätzlich zu den genannten Hauptkriterien kann zur Charakterisierung der IFN-gamma-Gruppe herangezogen werden, daß keine immunologische Kreuzreaktion mit den beiden anderen Gruppen erfolgt sowie ihre biologische Instabilität bei pH 2 im Gegensatz zu IFN-alpha und IFN-beta.

IFN-gamma wird nach Stimulation der Gesamtpopulation der Leukozyten, nach der Art der Präparation üblicherweise als buffy coats bezeichnet, durch Mitogene, Antigene oder spezifische Antikörper in einem komplexen Prozeß zusammen mit einer großen Anzahl weiterer Faktoren (Mediatoren) ausgeschüttet wie Interleukin 1 (IL 1), Interleukin 2 (IL 2), koloniestimulierender Faktor (CSF), migrationsinhibierender Faktor (MIF), Makrophagen aktivierender Faktor (MAF), Tumor nekrotisierender Faktor (TNF) Lymphotoxin (LT) und Fibroblastenwachstums-Faktor (FGF). Weitere Faktoren sind beschrieben von J. A. Georgiades et al. in Came, P. E. und Carter, W.A. (eds.), Interferons and Their Applications, Springer Verlag, 1984, und von Waksman, B. H. in Cohen, S. et al. (eds.), Biology of the Lymphokines, Academic Press Inc., 1979.

Diejenigen Faktoren, die von der Untergruppe der Lymphozyten gebildet werden, bezeichnet man allgemein als Lymphokine. Zu dieser Gruppe wird auch IFN-gamma gerechnet, das hauptsächlich von T-Lymphozyten produziert wird. Die Syntheseleistung der IFN-gamma produzierenden T-Lymphozyten wird ihrerseits wieder von dem Vorhandensein der oben erwähnten Faktoren beeinflußt.

Weiterhin existieren etablierte bzw. transformierte Zellinien, die IFN-gamma konstitutiv produzieren, wie von N. Fujii et al. in J. Immunology 130, S. 1683-1686, 1983, und A. Zlotnik et al. in J. Immunology 131, S. 794-800, 1983, beschrieben.

Ein bevorzugtes Verfahren zur Gewinnung von humanem Roh-IFN-gamma wird nachfolgend beschrieben. Als Ausgangsmaterial dienen lymphozytenreiche Plasmafraktionen von Blutkonserven, sogenannte "buffy coats", die gepoolt und durch schonende Zentrifugation bei 600 - 800 g von Plasmaresten befreit werden. Die pelletierten Gesamtleukozyten werden in vorgewärmtem Medium mit einer Dichte von 5 Millionen Zellen/ml suspendiert. Die Zellsuspension wird in aliquots in geeigneten Kultivierungsgefäßen nach Zugabe eines Mitogens (z.B. Phythämagglutinin) und Phorbolester [z.B. Phorbolmyristylacetat (PMA)] bis zu 70 Stunden bei 37 Grad C auf einem Schüttler inkubiert. Die IFN-gamma-haltigen Kulturüberstände werden durch Zentrifugation gewonnen und können bis zur Weiterverwendung bei 4 Grad C gelagert werden. Üblicherweise enthalten die so gewonnenen Präparationen etwa 10.000 Internationale Referenzeinheiten (I.E.) IFN-gamma pro ml.

Der IFN-gamma-Gehalt wird durch einen CPE-Reduktionstest in geeigneten Indikatorzellen (z.B. WISH) mit einem Testvirus (z.B. EMC der Maus) gegen den Referenzstandard Gg 23-901-530 des National Institute of Health (USA) als antivirale Aktivität des Präparates bestimmt.

Weitere Methoden zur Gewinnung von IFN-gamma-Präparationen werden von Y.K. Yip et al. in Infection and Immunity 34 S. 131- 139, 1981 und in den US-PS 4,376,821 und 4,376,822 sowie 4,460,685 beschrieben.

Ein grundsätzlich anderer Weg zur Gewinnung von humanen IFN-gamma-Präparaten wird mit der Einschleusung des humanen Strukturgens mit Hilfe geeigneter Vektoren in heterologe Wirtszellen beschritten. Diese rekombinanten Gene werden konstitutiv oder nach Zugabe spezifischer Induktoren von der Wirtszelle exprimiert. Auch wenn es sich bei dem Strukturgen um eine eindeutig definierte Sequenz aus Nukleotiden handelt, die zwingend zur Synthese einer daraus ableitbaren Aminosäuresequenz führt, ist das IFN-gamma-Molekül des mit Hilfe der Wirtszelle hergestellten Präparates nicht notwendigerweise mit einem natürlich vorkommenden IFN-gamma identisch. Diese Möglichkeit der Variation unter Erhaltung der spezifischen Wirkung durch "post processing" wird durch das sogenannte Proteindesign erweitert, durch das das Strukturgen durch chemische bzw. biochemische Vollsynthese oder Modifikation verändert werden kann.

Als Wirtszellen zur Aufnahme des Humangens können außer Bakterien (z.B. E.coli wie von G. Simons et al. in Gene 28, S. 55-64, 1984, beschrieben) auch Hefen (z.B. Saccharomyces cerevisiae, wie von R. Derynck et al. in Nucleic Acids Research 11, S. 1819-1837, 1983, beschrieben) oder eukaryotische Zellen (wie z.B. Chinese Hamster Ovary Cells, wie von S. J. Scahill et al. in Proc. Nat. Acad. Sci. USA 80, S. 4654-4658, 1983, Affenzellen, wie von P. W. Gray et al. in Nature 295, S. 503-508, 1982, beschrieben) dienen.

In einigen dieser Systeme reichert sich entweder IFN-gamma bis zu einer Konzentration von mehr als 100.000 I.E. pro ml in der Kulturflüssigkeit an oder es wird im Wirt selbst angereichert und stellt dort bis zu 25 Prozent des Proteingehalts der Zelle dar.

Die Anreicherung bzw. Reinigung der nach den vorstehend beschriebenen Verfahren erhaltenen IFN-gamma-Präparationen kann nach den folgenden Methoden einzeln oder in Kombination erfolgen:
1. Controlled Pore Glass (CPG) oder Silica-Gel;
2. Gelfiltration (z.B. AcA 54 oder Sephacel S 200);
3. Ionenaustauschchromatographie (CM-Sepharose oder Phosphocellulose oder DEAE-cellulose);
4. Affinitätschromatographie (Con A-Sepharose oder Poly-U-Sepharose oder Cu-Chelat-Sepharose);
5. Immunaffinitätschromatographie (Anti-IFN-gamma-Sepharose);
6. HPLC (z.B. mit Reversed Phase Materialien).

Entsprechende Verfahren wurden von Y.K. Yip et al. "Partial Purification and Characterization of Human Gamma (Immune) Interferon" in Proc. Nat. Acad. Sci. USA, 78, S. 1601-1605, 1981, oder von D. Novick et al., EMBO Journal 2, S. 1527-1530, 1983, oder in der DE-PS 3136166 A 1 beschrieben.

Durch geeignete Kombinationen ist eine Reinigung bis zur elektrophoretischen Homogenität möglich, z. Zt. liegt die dabei erreichte maximale spezifische Aktivität bei 100 - 200 Millionen I.E., während die beschriebenen Durchschnittswerte bei 10 und 50 Millionen I.E. liegen.

Die Interferone (IFN-alpha, IFN-beta, IFN-gamma) wurden bisher in sehr unterschiedlichen Dosen und Applikationsweisen bei Patienten therapeutisch eingesetzt. Ein- oder mehrmalig pro Woche verabreichte Dosen bis zu ca. 10 Millionen I.E. werden gemeinhin als niedrig, Dosen ab 20-50 Millionen I.E. als hoch bezeichnet.

In der Anfangszeit der therapeutischen Anwendung der Interferone standen aufgrund des Fehlens geeigneter biotechnologischer Herstellungsverfahren diese Substanzen lediglich in so geringen Mengen zur Verfügung, daß nur Dosen von weniger als 3 Millionen I.E. verabreicht werden konnten. Der Einsatz höherer Dosen ist beim IFN-alpha und IFN-beta seit Mitte der siebziger Jahre, beim IFN-gamma seit 1-2 Jahren möglich. Seither werden bei einer systemischen Behandlung Dosen von weniger als 3 Millionen I.E. in der Regel nur in Toleranz- und Toxizitätsprüfungen sowie in pharmakokinetischen Studien eingesetzt.

Für IFN-alpha und IFN-beta gilt, daß bei der systemischen Behandlung von Tumoren und Viruserkrankungen Dosen bis zu ca. 3 Millionen I.E. entweder unwirksam oder höheren Dosen unterlegen sind (Tumoren: Kirkwood, J.M. und Ernstoff, M.S.: J. Clin. Oncol. 2, 336-352, 1984; Billiau, A.: Contr. Oncol. 20, 251-269, 1984; Bonnem, E.M. und Spiegel, R.J.: J. Biol. Resp. Modif. 3, 580-598, 1984; Viruserkrankungen: Merigan, T.C. et al.: N. Engl. J. Med. 298, 981-987, 1978; Heidemann, E. et al. Dtsch. Med. Wschr. 107, 695-697, 1982; Levin, S.: Isr. J. Med. Sci. 19, 955-958, 1983; Billiau, A.: s.o., Greenberg, S.B. und Harmon, M.W., Armstrong, J.A. in Came, P.E. und Carter, W.A.: Interferons and Their Applications. Springer Verlag, Berlin 1984). Lediglich bei lokaler Behandlung (intratumorale, peritumorale, intraventrikuläre, intrathekale, intraläsionale, periläsionale oder intranasale Applikation) zeigen Dosen von weniger als 3 Millionen I.E. örtlich eine vergleichbare Wirksamkeit wie eine systemische Applikation höherer Dosen (Literatur s.o.).

Bei akuten Viruserkrankungen wie Herpes zoster hat sich eine Dosis von 0,5 Millionen I.E. IFN-alpha oder IFN-beta pro kg Körpergewicht und Tag bewährt, d.h. bei Erwachsenen täglich ca. 30 Millionen I.E. (Merigan, T.C.: s.o.; Heidemann, E. et al.: Onkologie 7, 210-212, 1984). Bei chronischen Viruserkrankungen wie der chronisch aktiven Hepatitis B werden in der Regel 3-10 Millionen I.E. IFN-alpha oder IFN-beta täglich oder 3mal wöchentlich gegeben (Müller, R. et al.: Z. Gastroenterologie 20, 105-109, 1982; Billiau, A.: s.o.; Levin, S.: s.o.).

Aufgrund von In-vitro-Untersuchungen gilt IFN-gamma als weniger antiviral wirksam als IFN-alpha oder IFN-beta (z.B. Munoz, A., Carrasco, L.: FEMS Microbiol. Letters 21, 105-111, 1984). Aus diesem Grunde wurde IFN-gamma für die systemische Behandlung von Viruserkrankungen bislang nicht verwendet.

Patienten mit Tumoren wurden bisher lediglich in Rahmen von Phase-I-Studien behandelt, so daß eine Aussage über eine therapeutische Wirksamkeit von IFN-gamma bei diesen Erkrankungen nicht möglich ist (Yamazaki, S.: Jpn. J. Med. Sci. Biol. 37, 209-223, 1984; Sherwin, S.A. et al.: J. Biol. Resp. Modif. 3, 599-607, 1984; Guttermann, J.U. et al.: Cancer Res. 44, 4164-4171, 1984; Niederle et al. in Kirchner, H. und Schellekens H.: The Biology of the Interferon System 1984, Elsevier, Amsterdam 1985.)

Bei Psoriasis, allergischen Erkrankungen, M. Crohn, Amyotropher Lateralsklerose und Schmerzen wurde IFN-gamma bisher nicht eingesetzt.

Von Sherwin wird in "Interferon : In vivo and clinical studies" Bd. 4, (1985) Elsevier, Amsterdam, S. 321-322, über klinische Versuche der Phase I mit teilweise gereinigtem natürlichem Interferon gamma berichtet und ausdrücklich festgestellt, daß Ergebnisse klinischer Versuche mit Interferon gamma (rekombiniert human) nicht vorliegen.

Wie aus der (a.a.O.S. 321) zitierten Originalarbeit von Sherwin et al. (1984) hervorgeht, konnte in diesen Studien bei keinem Patienten eine Tumorregression festgestellt werden. Diesen Dokumenten konnte der Fachmann nur die Lehre entnehmen, daß bei Verabreichung von 0,2, 0,5 und 1,0 x 10⁶ I.E./m² natürlichem Interferon gamma zweimal wöchentlich während 4 bis 6 Wochen keine antitumorale Wirkung in Phase 1 festzustellen ist und eine antivirale Aktivität erst bei Dosen oberhalb von 2 x 10⁶ I.E./m² meßbar war. Da der Fachmann weiß, daß eine antivirale Aktivität im Serum keinen Rückschluß auf eine antivirale therapeutische Wirkung zuläßt, zudem von Sherwin ausdrücklich darauf hingewiesen wird (S. 322), daß die beobachteten Wirkungen auch durch andere anwesende biologisch aktive Substanzen mitverursacht sein konnten, war diesem Stand der Technik die erfindungsgemäße Lehre nicht entnehmbar.

In der GB-A-2 040 292 wird im wesentlichen nur die Herstellung von Interferon gamma enthaltenen Präparationen beschrieben, wobei die mit diesen Präparationen durchgeführten Versuche nur in vitro und im Nacktmausmodell durchgeführt wurden. Die berichteten Ergebnisse lassen keine Schlußfolgerung auf eine Wirksamkeit beim Menschen zu, vor allem nicht darauf, daß niedrige Dosen therapeutisch wirksamer sind als hohe.

In der EP-A 80 032 wird lediglich die topische Applikation von natürlichem lnterferon alpha zur Behandlung von Herpes labialis und Herpes genitalis beschrieben.

Die EP-A-87 686 betrifft im wesentlichen die Reinigung von humanem Interferon gamma, wobei über einen möglichen therapeutischen Einsatz nur spekulativ berichtet und ein bevorzugter Dosisbereich zwischen 10 und 100 Millionen I.E. (vgl. S. 9, Z. 6-9) vorgeschlagen wird.

Die EP-A 77 063 betrifft ausschließlich die topische Applikation von Dermatika, die als Wirksubstanz Interferon alpha enthalten.

Die EP-A 119 757 betrifft neue Guaninderivate und pharmazeutische Zubereitungen, die zusätzlich Interferon alpha und/oder Interferon gamma enthalten. Eine Hochrechnung der in Tabelle II (vgl. S. 11) für Interferon gamma angegebenen Gehaltsmengen führt zu Werten >16 x 10⁶ I.E./60 kg.

In eigenen klinischen Studien zur therapeutischen Wirksamkeit von IFN-gamma wurden Patienten mit unterschiedlichen Erkrankungen behandelt. Um die optimal wirksame Dosis von IFN-gamma zu ermitteln, wurden Dosen von 50.000 I.E. bis zu 100 Millionen I.E. ein- oder mehrmalig pro Woche systemisch verabreicht. Bei diesen Studien zeigte sich überraschend, daß IFN-gamma im Gegensatz zu IFN-alpha oder IFN-beta bei sehr verschiedenen Erkrankungen therapeutisch wirksam war und die effektivste Dosis von IFN-gamma bei allen Erkrankungen in dem sehr niedrigen Bereich von 100 000 bis 2 Millionen I.E. bzw. etwa 10 - 200 µg lag. Bei Patienten, die mit diesen niedrigen Dosen behandelt wurden, war häufiger eine Wirkung von IFN-gamma festzustellen als bei Patienten, die höhere Dosen erhielten.

Insbesondere überraschte die Wirksamkeit bei verschiedenen Viruserkrankungen wie chronisch aktive Hepatitis B (Beispiel 2), Herpes zoster (Beispiele 3) und Kondylomen (Beispiel 4). IFN-gamma zeigte bei diesen Erkrankungen nämlich trotz der geringen In-vitro-Aktivität nicht nur in gleicher Dosierung wie IFN-alpha oder IFN-beta, sondern sogar in wesentlich niedrigerer Dosierung als die beiden anderen Interferontypen einen therapeutischen Effekt.

Ebenfalls unerwartet war IFN-gamma wirksam bei folgenden Erkrankungen: Asthma (Beispiel 5), M. Crohn (Beispiel 6) und Amyotropher Lateralsklerose (Beispiel 7).

Für die systemische Behandlung kann rekombinantes IFN-gamma wie folgt dosiert werden: intravenös als Bolus oder über mehrere Minuten, Stunden, Tage oder Wochen, intramuskulär und/oder subkutan. Bei allen Applikationsformen können auch die dem Fachmann bekannten Depotformen verwendet werden. Eine Dosis von 100 000 I.E. (ca. 10 µg) bis 2 Millionen I.E. (ca. 200 µg) kann bei wiederholter Gabe wie folgt verabreicht werden:
a) an aufeinanderfolgenden Tagen
b) alle 2 bis 6 Tage
c) einmal pro Woche
d) alle 2 bis 4 Wochen
e) einmal pro Monat
f) jedesmal bei Verschlechterung des Krankheitsbildes.

Die Dosis von 100 000 - 2 Millionen I.E. bezieht sich auf einen Patienten von 60 kg Körpergewicht und 170 cm Körpergröße, entsprechend 1,74 qm Körperoberfläche (Dubois u. Dubois, Arch. intern. Med. 17, 863, 1916). Entsprechend wird für den einzelnen Patienten die individuelle Dosis ermittelt, d.h. 0,06 - 1,2 x 10⁶ I.E. pro m² Körperoberfläche.

Zur Steigerung der Effektivität der Interferon-gamma-Präparationen können folgende Substanzen zusätzlich gegeben werden:
a) Verabreichung von anderen Interferonen und/oder anderen von Leukozyten gebildeten bzw. durch gentechnologische Verfahren hergestellten Zell-Mediatoren wie IL 1, IL 2, CSF, MIF, MAF, TNF, LT und FGF;
b) Verabreichung von in der Therapie von Viruserkrankungen, Psoriasis und allergischen Erkrankungen bisher verwendete Substanzen wie Alkylanzien, Folsäureantagonisten, Antimetabolite des Nukleinsäurestoffwechsels, Spindelgifte,
   Antibiotika, Immuntherapeutika, Pyrimidinanaloga, Purinnukleoside, Amine, Triazolnukleoside, Kortikoide, Kalzium, Antihistaminika, Retinoide, lichtsensibilisierende Substanzen, Inhibitoren der Lipoxygenase bzw. Cyclooxygenase, Fumarsäure und deren Salze, Analgetika, Psychopharmaka, Lokalanästhetika, Spasmolytika, Antirheumatika, Kalzium-Antagonisten und Betablocker.
   Detaillierte Angaben hierzu werden gemacht und weitere Substanzen sind beschrieben von UICC (Hrsg.) in Klinische Onkologie, Springer Verlag, 1982, von J. Fischer (Hrsg.) in Taschenbuch der Onkologie, Urban und Schwarzenberg, 1983, von E. De Clercq in Biochem. J. 205, S. 1-13, 1982, von K.G. Nicholson in Lancet ii, S. 503-506, S. 562-564, S. 617-621, S. 677-682, S. 736-739, 1984, von Braun-Falco, O. et al.: Dermatologie und Venerologie. 3. Aufl. Springer Verlag, Berlin 1984, von Nietsch, P.: Schmerztherapie aktuell, Folge 1-10, medwelt 35, 1984, von Senn, H.J. und Glaus, A.: medwelt 35, 1235-1240, 1984 und von Foley, K.M.: N. Engl. J. Med. 313, 84-95, 1985.
c) Radiologische Maßnahmen wie Bestrahlung oder Einführen radioaktiver Substanzen wie beschrieben von UICC (Hrsg.) in Klinische Onkologie, Springer Verlag, 1982.

Zur Herstellung der jeweiligen Darreichungsform werden die dem Fachmann bekannten Hilfsstoffe verwendet, z.B. Serumeiweißstoffe wie humanes Serumalbumin, Puffersubstanzen wie Phosphate, Glycin, Sorbinsäure, Kaliumsorbat, Partialglyceridgemische gesättigter Pflanzenfettsäuren, Wasser sowie Salze bzw. Elektrolyte wie Protaminsulfat, Dinatriumhydrogenphosphat, Kaliumhydrogenphosphat, Natriumchlorid und Zn-Salze.

### BEISPIEL 1

Patient:
W. R., männlich, 74 J., 64 kg, 170 cm
Diagnose:
Hypernephrom mit cutanen, pulmonalen und ossären Metastasen Psoriasis vulgaris
Substanz:
humane IFN-gamma-Präparation aus E.coli und Difluoromethylornithin (DFMO)
Applikationsweise:
IFN-gamma intravenös, DFMO oral Therapieschema:
IFN-gamma zunächst täglich 50 µg über 10 Tage, dann täglich 100 µg über 7 Tage, DFMO täglich 3 x 4 g
Ergebnis
: Am 7. Tag gingen die Schmerzen zurück, am 14. Tag war der Patient vollkommen schmerzfrei, Ab dem 12. Tag bildete sich die Psoriasis vulgaris zurück und war bei Therapieende annähernd komplett verschwunden.
Bei der Entlassung des Patienten nach 17 Theräpietagen war eine partielle Rückbildung der cutanen und der Lebermetastasen nachweisbar. Der Allgemeinzustand des Patienten hatte sich deutlich gebessert..

### BEISPIEL 2

Patient:
M. K., weiblich, 39 J.
Diagnose:
chronisch aktive Hepatitis B
Substanz:
humane IFN-gamma-Präparation aus E. coli
Applikationsweise:
intravenös
Therapieschema:
5mal pro Woche über 10 Wochen: Die Dosis wurde einschleichend von anfangs 0,05 x 10 ⁶ auf 2,0 x 10⁶ I.E. gesteigert.
Ergebnis:
Unter der Therapie sank die DNAP von 17 422 auf 5 546 ab.

### BEISPIEL 3

Patient:
T. S., männlich, 51 J.
Diagnose:
Zoster oticus mit Facialisparese
Substanz:
humane IFN-gamma-Präparation aus E.coli
Applikationsweise:
intramuskulär
Therapieschema:
100 µg 2mal pro Tag über 5 Tage
Ergebnis:
Nach 5 Tagen Therapie war der Zoster nahezu abgeheilt, und die Funktion des Nervs hatte sich normalisiert.

### BEISPIEL 4

Patient:
N. N., weiblich, 20 J.
Diagnose:
Condylomata acuminata
Substanz:
humane IFN-gamma-Präparation aus E.coli
Applikationsweise:
subcutan
Therapieschema:
täglich 200 µg über 7 Tage
Ergebnis:
Innerhalb von 3 Wochen nach Ende der Behandlung waren die Kondylome nahezu vollständig verschwunden.

### BEISPIEL 5

Patient:
I K., weiblich, 56 J.
Diagnose:
Asthma bronchiale
Substanz:
humane IFN-gamma-Präparation
Applikationsweise:
subcutan
Therapieschema:
3 Injektionen im Abstand von 3 Tagen mit jeweils 100 µg
Ergebnis:
Nach der Therapie war die Atemnot der Patientin deutlich verringert. Die Medikation mit Kortikosteroiden war nicht mehr notwendig. Die Gabe von Theophyllin konnte in der Dosis auf die Hälfte reduziert werden.

### BEISPIEL 6

Patient:
I. B., männlich, 44 J., 57 kg
Diagnose:
M. Crohn
Substanz:
humane IFN-gamma-Präparation aus E. coli
Applikationsweise:
intramuskulär oder intravenös
Therapieschema:
3-4 mal pro Woche über 8 Wochen: Die Dosierung wurde einschleichend von anfangs 0,02 auf 0,5 x 10⁶ I.E. gesteigert.
Ergebnis:
Der Crohnindex war durch die Therapie von 290 auf 59 gefallen. (In den Crohnindex gehen mehrere Faktoren ein u.a. Anzahl der Stühle, Bauchschmerzen, Allgemeinbefinden, Gewicht. Diese werden mit Punkten bewertet: Je höher die Punktzahl ist, desto ausgeprägter ist die Erkrankung).

### BEISPIEL 7

Patient:
W. W., männlich, 61 J., 65 kg, 175 cm
Diagnose:
Amyotrophe Lateralsklerose
Substanz:
humane IFN-gamma-Präparation aus E coli.
Applikationsweise:
intramuskulär
Therapieschema:
3mal pro Woche über 4 Wochen, Dosis von 0,1 x 10⁶ auf 1 x 10⁶ I.E. steigernd. Danach 1mal pro Woche 0,5 x 10⁶ I.E. über 4 Wochen.
Ergebnis:
Unter Therapie besserten sich die Funktionen der oberen Extremität. Die Hände konnten wieder gebeugt und gestreckt werden, auch waren mäßige Streckungen und Beugungen sowie geringfügige Rotationen im Ellenbogengelenk möglich. Beim Schultergelenk war wieder eine geringe Elevation möglich.

## Patentansprüche

1. Verwendung von Interteron gamma (rekombiniert human) zur Herstellung von Präparationen zur systemischen Behandlung akuter Viruserkrankungen, enthaltend 100 000 bis 2 Millionen Internationale Referenzeinheiten (I.E.) (entsprechend ca. 10 bis 200 µg) als Tagesdosis, bezogen auf einen erwachsenen Patienten von etwa 60 kg Körpergewicht und 1,7 m² Körperoberfläche.

2. Verwendung von Interferon gamma (rekombiniert human) nach Anspruch 1 zur Herstellung von Präparationen zur Behandlung chronischer Viruserkrankungen.

3. Verwendung von Interferon gamma (rekombiniert human) nach Anspruch 1 zur Herstellung von Präparationen zur Prophylaxe von akuten und chronischen Viruserkrankungen.

4. Verwendung von Interferon gamma (rekombiniert human) nach Anspruch 2 zur Herstellung von Präparationen zur Behandlung von durch humane Papillomviren (HPV) bedingte Erkrankungen.

5. Verwendung von Interferon gamma (rekombiniert human) nach Anspruch 4 zur Herstellung von Präparationen zur Behandlung von Condylomata acuminata.

6. Verwendung von Interferon gamma (rekombiniert human) zur Herstellung von Präparationen zur systemischen Behandlung psoriatischer Erkrankungen, enthaltend 100 000 bis 2 Millionen Internationale Referenzeinheiten (I.E.) (entsprechend ca. 10 bis 200 µg) als Tagesdosis, bezogen auf einen erwachsenen Patienten von etwa 60 kg Körpergewicht und 1,7 m² Körperoberfläche.

7. Verwendung von Interteron gamma (rekombiniert human) nach Anspruch 6 zur Herstellung von Präparationen zur Behandlung von Psoriasis vulgaris.

8. Verwendung von Interferon gamma (rekombiniert human) zur Herstellung von Präparationen zur systemischen Behandlung von allergischen Erkrankungen, enthaltend 100 000 bis 2 Millionen Internationale Referenzeinheiten (I.E.) (entsprechend ca. 10 bis 200 µg) als Tagesdosis, bezogen auf einen erwachsenen Patienten von etwa 60 kg Körpergewicht und 1,7 m² Körperoberfläche.

9. Verwendung von Interferon gamma (rekombiniert human) nach Anspruch 8 zur Herstellung von Präparationen zur Behandlung von Asthma bronchiale.

10. Verwendung von Interferon gamma (rekombiniert human) zur Herstellung von Präparationen zur systemischen Behandlung von Morbus Crohn, enthaltend 100 000 bis 2 Millionen Internationale Referenzeinheiten (I.E.) (entsprechend ca. 10 bis 200 µg) als Tagesdosis, bezogen auf einen erwachsenen Patienten von etwa 60 kg Körpergewicht und 1,7 m² Körperoberfläche.

11. Verwendung von Interferon gamma (rekombiniert human) zur Herstellung von Präparationen zur systemischen Behandlung der Amyotrophen Lateralsklerose, enthaltend 100 000 bis 2 Millionen Internationale Referenzeinheiten (I.E.) (entsprechend ca. 10 bis 200 µg) als Tagesdosis, bezogen auf einen erwachsenen Patienten von etwa 60 kg Körpergewicht und 1,7 m² Körperoberfläche.

12. Verwendung von Interferon gamma (rekombiniert human) zur Herstellung von Präparationen nach einem der Ansprüche 1 bis 11, dadurch gekennzeichnet, daß die Interferon gamma enthaltende Präparation zusätzlich andere Interferone und/oder andere durch Leukozyten gebildete bzw. durch gentechnologische Verfahren hergestellte Zell-Mediatoren enthält.

13. Verwendung von Interferon gamma (rekombiniert human) zur Herstellung von Präparationen nach einem der Ansprüche 1 bis 12, dadurch gekennzeichnet, daß diese Präparation zusätzlich andere bisher in der Therapie von und Viruserkrankungen, Psoriasis Asthma verwendete Substanzen enthält.

14. Verwendung von Interferon gamma (rekombiniert human) nach einem der Ansprüche 1 bis 13 zur Herstellung von Präprationen in einer intravenös, intramuskulär oder subkutan zu verabreichenden Applikationsform.

15. Verwendung von Interferon gamma (rekombiniert human) nach Anspruch 14 zur Herstellung von dem Fachmann für Polypeptidtherapeutika bekannten Deportformen.

## Claims

1. Use of interferon gamma (recombinant human) for the production of preparations for the systemic treatment of acute viral diseases, containing 100,000 to 2 million international reference units (I.U.) (corresponding to about 10 to 200 µg) as daily dose based on an adult patient of about 60 kg body weight and 1,7 m² body surface area.

2. Use of interferon gamma (recombinant human) according to Claim 1 for the production of preparations for the treatment of chronic viral diseases.

3. Use of interferon gamma (recombinant human) according to Claim 1 for the production of preparations for the prophylaxis of acute and chronic viral diseases.

4. Use of interferon gamma (recombinant human) according to Claim 2 for the production of preparations for the treatment of diseases caused by human papilloma-viruses (HPV).

5. Use of interferon gamma (recombinant human) according to Claim 4 for the production of preparations for the treatment of condylomata acuminata.

6. Use of interferon gamma (recombinant human) for the production of preparations for the systemic treatment of psoriatic diseases, containing 100,000 to 2 million international reference units (I.U.) (corresponding to about 10 to 200 µg) as daily dose based on an adult patient of about 60 kg body weight and 1,7 m² body surface area.

7. Use of interferon gamma (recombinant human) according to Claim 6 for the production of preparations for the treatment of psoriasis vulgaris.

8. Use of interferon gamma (recombinant human) for the production of preparations for the systemic treatment of allergic diseases, containing 100,000 to 2 million international reference units (I.U.) (corresponding to about 10 to 200 µg) as daily dose based on an adult patient of about 60 kg body weight and 1,7 m² body surface area.

9. Use of interferon gamma (recombinant human) according to Claim 8 for the production of preparations for the treatment of bronchial asthma.

10. Use of interferon gamma (recombinant human) for the production of preparations for the systemic treatment of Crohn's disease, containing 100,000 to 2 million international reference units (I.U.) (corresponding to about 10 to 200 µg) as daily dose based on an adult patient of about 60 kg body weight and 1,7 m² body surface area.

11. Use of interferon gamma (recombinant human) for the production of preparations for the systemic treatment of amyotrophic lateral sclerosis, containing 100,000 to 2 million international reference units (I.U.) (corresponding to about 10 to 200 µg) as daily dose based on an adult patient of about 60 kg body weight and 1,7 m² body surface area.

12. Use of interferon gamma (recombinant human) for the production of preparations according to any of Claims 1 to 11, characterized in that the preparation containing interferon gamma additionally contains other interferons and/or other cell mediators formed by leucocytes or produced by genetic engineering processes.

13. Use of interferon gamma (recombinant human) for the production of preparations according to any of Claims 1 to 12, characterized in that this preparation additionally contains other substances used to date in the therapy of tumours, viral diseases, psoriasis, asthma and pain.

14. Use of interferon gamma (recombinant human) according to any of Claims 1 to 13 for the production of preparations in a form for intravenous, intramuscular or subcutaneous administration.

15. Use of interferon gamma (recombinant human) according to Claim 14 for the production of depot forms known to the expert for polypeptide therapeutics.

## Revendications

1. Utilisation d'interféron-gamma (recombiné humain) pour produire des préparations destinées au traitment systématique de maladies à virus aigües, contenant de 100 000 à 1 millions d'unités de référence internationale (U.I.) (correspondant environ à 10 jusqu'à 200 µg) comme dose journalière, rapporté à un patient adulte des poids 60 kg environ et de surface du corps 1,7 m².

2. Utilisation d'interféron-gamma (recombiné humain) selon la revendication 1 pour fabriquer des préparations destinées au traitement de maladies virales chroniques.

3. Utilisation d'interféron-gamma (recombiné humain) selon la revendication 1 pour fabriquer des préparations destinées à la prophylaxie de maladies virales aigües et chroniques.

4. Utilisation d'interféron-gamma (recombiné humain) selon la revendication 2 pour fabriquer des préparations destinées au traitement de maladies dues aux virus humains du papillome (V.H.P.).

5. Utilisation d'interféron-gamma (recombiné humain) selon la revendication 4 pour produire des préparations pour le traitement de "condylomata acuminata".

6. Utilisation d'interféron-gamma (recombiné humain) pour fabriquer des préparations pour le traitement systématique de maladies psoriatiques, contenant 100 000 à 2 millions d'unités de référence internationales (U.I.) (correspondant environ à 10 jusqu'à 200 µg) comme dose journalière, rapporté à un patient adulte de 60 kg environ et 1,7 m² de surface de corps.

7. Utilisation d'interféron-gamma ( recombiné humain) selon la revendication 6 pour produire des préparations pour le traitement de "psoriasis vulgaris".

8. Utilisation d'interféron-gamma (recombiné humain) pour produire des préparations, pour traitement systématique de maladies allergiques, contenant 100 000 à 2 millions d'unités de référence internationales (U.I) (correspondant environ à 10 jusqu'à 200 µg) comme dose journalière, rapporté à un patient adulte d'environ 60 kg et 1,7 m² de surface de corps.

9. Utilisation d'interféron-gamma (recombiné humain) selon la revendication 8 pour produire des préparations pour traitement de l'asthme bronchial

10. Utilisation d'interféron-gamma (recombiné humain) pour produire des préparations destinées au traitement systématique de la maladie de Crohn, contenant 100 000 à 2 millions d'unités de référence internationales (U.I.) (correspondant environ à 10 jusqu'à 200 µg) comme dose journalière, rapporté à un patient adulte d'environ 60 kg et 1,7 m² de surface de corps.

11. Utilisation d'interféron-gamma (recombiné humain) pour produire des préparations destinées au traitement systématique de la sclérose latérale amyotrophe, contenant 100 000 à 2 millions d'unités de référence internationales (U.I.) (correspondant environ à 10 jusqu'à 200 µg) comme dose journalière, rapporté à un patient adulte d'environ 60 kg et de 1,7 m² de surface de córps.

12. Utilisation d'interféron-gamma (recombiné humain) pour la production de préparation selon l'une quelconque des revendications 1 à 11, caractérisée en ce que la préparation contenant l'interféron-gamma contient en plus d'autres interféron et/ou d'autres médiateurs de cellules formés par des leucocytes ou produites par des procédés genotechnologiques.

13. Utilisation d' interféron-gamma (recombiné humain) pour produire des préparations selon l'une quelconque des revendications 1 à 12, caractérisée en ce que comme préparation contient additionnellement d'autres substances utilisées jusqu'à maintenant dans la thérapie de tumeurs, maladies à virus, psoriasis, asthme et douleurs.

14. Utilisation d'interféron-gamma (recombiné humain) selon l'une des revendications 1 à 13 pour produire des préparations à administrer sous forme d'application interveineuse, intramusculaire ou sous-cutanée.

15. Utilisation d'interféron-gamma (recombiné humain) selon la revendication 14 pour produire de formes de départ connues du spécialistes pour la thérapeutie polypeptide.
